# EUROPEAN PATENT APPLICATION

(11) **EP 4 768 563 A1**
(43) Date of publication of application: **01.07.2026**
(21) Application number: 24383458.7
(22) Date of filing: 24.12.2024
(51) Int. Cl.: C12M 1/00, C12M 1/40, B01L 3/00

(54) **ENZYME SCREENING BIOREACTOR AND PLATFORM CONTAINING A PLURALITY OF THEM**

(71) Applicant: Acondicionamiento Tarrasense, 08225 Terrassa (Barcelona) (ES)
(72) Inventor: Cabot Canyelles, Joan Marc, Terrassa (ES); Grinyte, Ruta, Terrassa (ES); Azizian, Pooya, Terrassa (ES)
(74) Representative: Pons IP

(57) **Abstract**

The present invention relates to an enzyme screening bioreactor (10) comprising an inlet channel (1) configured for the introduction of a micropipette containing elements like microparticles (7) and at least one reagent, a reaction area in direct communication with the inlet channel (1) configured to contain the elements introduced through the inlet channel (1), an outlet channel (6) configured for the evacuation of the air contained in the bioreactor (10), and a detection area (4) comprising an observation window (5), wherein the screening bioreactor (10) comprises a separation site (3) in the form of an obstruction to separate the reaction area (2) from the detection area (4) that comprises a channel. The invention also refers to a high-throughput enzyme screening platform (20) comprising a plurality of the mentioned enzyme screening bioreactors (10).

## Description

### OBJECT OF THE INVENTION

The present invention relates to a screening platform, mainly focused on microfluidic activities, configured to carry out catalytic (enzymatic) assays and quantify their activities so as a high-throughput platform comprising a plurality of the screening bioreactors.

### BACKGROUND OF THE INVENTION

The present invention aims to swiftly select enzyme variants from large collections and libraries.

Conventional enzyme production yields small amounts, and lab methods often require significant enzyme and substrate quantities, along with costly equipment and laborious sample prep by skilled personnel. Enzyme stability is also a common issue, complicating lengthy reactions. Lab-on-a-Chip systems integrate microfluidic and biosensing technology for miniaturized detection. Enzymes can be immobilized in these systems, offering rapid reactions, enhanced stability, reduced autolysis, and user-friendliness. These systems excel over traditional biocatalysis monitoring methods due to lower energy use, rapid heat exchange, efficient mass transfer, and superior repeatability.

### DESCRIPTION OF THE INVENTION

The present invention relates to a screening bioreactor, mainly focused on microfluidic activities, configured to carry out catalytic (enzymatic) assays and quantify their activities.

The screening bioreactor comprises the following parts:
- an inlet: channel configured for the introduction of microparticles and at least one reagents inside the bioreactor with a micropipette. It has a cone-like shape to properly adapt to the micropipette tip.
- a reaction area, configured as a channel, where the microparticles introduced are contained.
- a separation site in the form of an obstruction with the functionality of keeping the microparticles trapped and preventing them from passing from the reaction area to the detection area.
- an observation window located in the detection area in the form of a transparent area where the measurement and quantification is performed.
- an outlet channel, wider than the inlet channel, configured for the venting of the air that might be trapped inside the bioreactor as well as to allow the washing of both the microparticles and the observation window.

The main feature of the invention relates to the configuration of the screening bioreactor, which comprises the reaction area, where the functionalized microparticles trapped, and the detection area, located in a separated section, to keep the microparticles reusable.

The reaction area is the zone where the functionalized microparticles, with a dimension of 100-300 micron (µm) with enzymes, or other immobilized molecules, can be trapped.

The detection area comprises an observation window, a transparent zone through which analysis can be considered. In this regard, measurement and quantification (e.g., colorimetric reaction) produced by enzyme can be recorded.

The reaction area is separated from the detection area through the separation site, which comprises a channel with an opening smaller than 100 micron which avoids the cited microparticles to go through, offering high mass transfer as a result of high surface to volume ratio. It provides a short diffusion path, while the flow is non-turbulent and highly ordered.

In this regard, functionalized microparticles with enzymes or other immobilized molecules may be trapped in the reaction area of the bioreactor.

The bioreactor may be used at least ten times without any activity loss or leaching phenomena of the microparticles.

Moreover, the screening bioreactors require a small amount of enzymes to work.

The invention also refers to a screening platform comprising a plurality of the mentioned screening bioreactors.

### DESCRIPTION OF THE DRAWINGS

For a better understanding of the present invention, the figures listed below are included in this specification.
Figure 1 represents an enzyme screening bioreactor according to the invention.
Figure 2 represents a screening platform comprising a plurality of enzyme screening bioreactor
Figure 3 represents a section of the screening platform of figure 2 so that the plurality of enzyme screening bioreactor may be seen.

The following is a list of the references used in the figures to facilitate their follow-up:
1. Inlet channel.
2. Reaction area.
3. Separation site.
4. Detection area.
5. Observation window.
6. Outlet channel.
7. Microparticles.
10. Bioreactor.
20. Platform.

### PREFERRED EMBODIMENT OF THE INVENTION

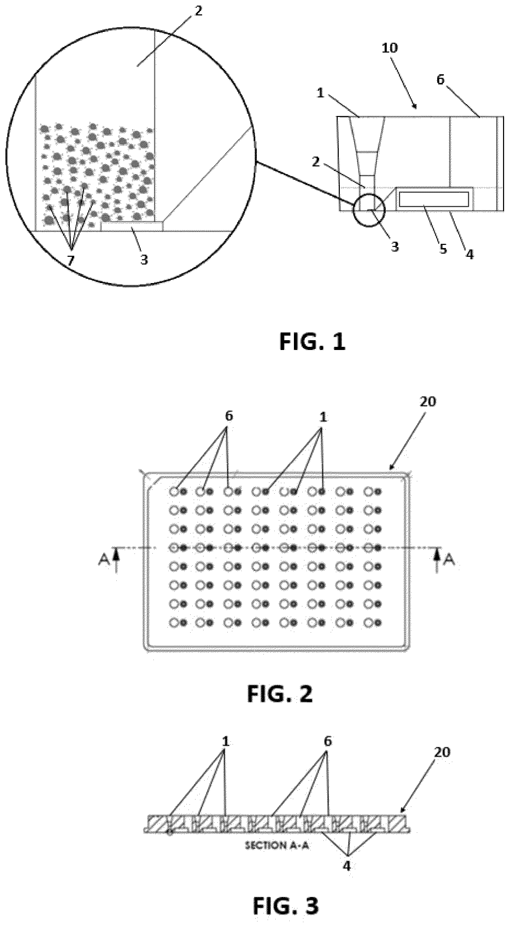

The present invention relates to an enzyme screening bioreactor (10) and a screening platform (20) comprising a plurality of the screening bioreactors (10).
an embodiment of the enzyme screening bioreactor (10) of the invention is represented in figure 1. It may be seen that it comprises the following parts:
- An inlet: channel (1).
- A reaction area (2).
- A separation site (3).
- A detection area (4)
- An observation window (5).
- An outlet channel (6).

The inlet: channel (1) is used to introduce the microparticles (7) that might be needed inside the bioreactor (10), including any reagent. It has a cone-like shape to properly adapt to the tip of a micropipette used to introduce the microparticles (6).

The reaction area (2) is configured as a channel in direct communication with the inlet channel (1) where the microparticles (7) are contained after introduced through the inlet channel (1).

The separation site (3) is configured in the form of an obstruction in the reaction area (2) that keeps the microparticles (7) retained.

The detection area (4) is the place where the measurement and quantification of the microparticles (7) is performed. It comprises an observation window (5) for the analysis.

The outlet channel (6) is wider than the inlet channel and is configured for the evacuation of the air that might be trapped inside the bioreactor (10) as well as to allow the washing of both the microparticles (7) and the observation window (5).

Figure 2 represents an enzyme screening platform (20) featuring up to 64 of the screening bioreactors (10) shown in figure 1.

Figure 3 shows the configuration of the platform (20) more clearly.

The screening bioreactor (10) of the invention allows a variety of commercially available microparticles (7) with different enzyme immobilization strategies without losing its activity after immobilization. After inserting the microparticles (7), the bioreactor (10) is stable and can be used over ten times for different types of analysis. At the time of changing the enzymes, contrary to the conventional systems, the bioreactor (10) is washable and reusable to avoid plastic waste and environmental pollution.

The screening process is based on the entrapment of the enzyme-functionalised microparticles (7) in the reaction area (2).

The enzyme is previously mixed with iminodiacetic functionalized particles. By his-tag enzyme reactive groups, enzyme binds to the surface of the microparticles (7). Methacrylic polymer matrix particles with a diameter of 100-300 µm are trapped in the bioreactor (10).

The entrapment of the microparticles (7) inside the bioreactor (10) is achieved by varying the channel height of the separation site (3), creating a constriction in the bioreactor (10) that prevents microparticles (7) located in the reaction area (2) from advancing to the detection area (4) while letting the reagent pass through.

For validation of the bioreactor (10), functionalized particles (4 µL of particles 2.4 mg) were trapped in the bioreactor (10) at a microfluidic section. 60 µL of enzymatic substrate was injected with automatic micropipettes, that offers to fill 8 inlet channels (1) at the same time. The bioreactor (10) was tested with commercial plate reader TECAN INFINITE^{®}. The absorbance changes against time were measured and enzymatic activity of model enzyme was detected.

Compared with commercially available multiwell plates NUNC^{®}, COSTAR^{®}, CORNINGO etc., using the same type of enzyme, the bioreactor (10) of the invention uses less volume of the reagent. Moreover, the same bioreactor (10) can be used over ten times, after following a washing procedure of the bioreactor (10) with Phosphate buffer saline after every test. Finally, the platform (20) is also reusable after being immersed in a fluid with 70 % of ethanol for 1 hour, removing the adhesive and being replaced by another one. The development of the screening bioreactor (10) of the invention, so as the platform (20), involves particle selection, manufacturing optimization and screening of enzyme activities. The initial model was originally based on packed-bed microfluidics using the horseradish peroxidase as accessory enzyme that is required for the development of a final colorimetric product and a commercially available laccase as oxidoreductase model enzyme.

The final system tested on enzymes where small amounts of polyhistidine tagged oxidases - namely alditol oxidase and eugenol oxidase - were immobilized by metal affinity binding through iminodiacetic functionality on microparticles with a high surface/volume ratio. The particles were successfully loaded on a microfluidic-based and parallelized in a multi-well plate format manufactured using the engineering plastic polymethyl methacrylate (PMMA, also known as acrylic) material and computer numerical control (CNC) machining technology. The functionality of the system was measured inside the 3D microfluidic layout by monitoring a change in colour due to the catalysed oxidation of chromogenic dyes by HRP. Moreover, the results achieved from the definitive microfluidic screening platform (20) were presented, where, enzymatic activity and kinetics can be determined, reused several times for replicates or even under various conditions such as pH and temperature. Reusability of the enzymatic bioreactor (10) can be done for at least one month without any activity lost, and enzymes can be reloaded at will.

To validate the microfluidic device for enzyme screening, many aspects were considered and improved with respect to more functional and user-friendly devices.

In order to obtain the final functional multi-bioreactor platform (20), several designs were generated. The first configuration featured seven horizontal, straight channels with different heights that was conceived to determine the best microparticles packing dimensions. In this early design, the loading of enzyme-functionalised microparticles was done by hand - either with a syringe or with a micropipette - but it required tubes and pumps to fulfil its intended purpose.

This pump-driven device was next modified into a more complex three-dimensional microfluidic layout already resembling the final configuration. Then the inlets and outlets were changed to allow for a manual operation by pipetting, being user-friendliness a key feature of this device, like regular well plates. The successive versions that followed included a number of modifications to improve the flow, diffusion, and other fluidic parameters inside the bioreactors (10) and to make it more user-friendly.

Once the fluidic behaviour was solid, the last step in the development of the microfluidic device was integrating the bioreactors (10) in a multiwell plate format for screening enzymes in parallel and compatibility with any commercial plate reader. Since a 384-well commercial plate was used as a base model for this microfluidic plate, some challenges had to be faced such as adapting the size of each of the small bioreactors (10) or units to the number of single wells, as well as the distance between the inlets of the units to make the plate compatible with commercial multichannel micropipettes. The last and definitive design contains 64 bioreactors (10).

The final plate was then manufactured in PMMA by CNC-machining technology for better optical properties and surface finish (figure 3).

## Claims

1. An enzyme screening bioreactor (10) comprising:
- an inlet channel (1) configured for the introduction of a micropipette containing elements like microparticles (7) and at least one reagent,
- a reaction area in direct communication with the inlet channel (1) configured to contain the elements introduced through the inlet channel (1),
- an outlet channel (6) configured for the evacuation of the air contained in the bioreactor (10),
- a detection area (4) comprising an observation window (5),
**characterized in that:**
the enzyme screening bioreactor (10) comprises a separation site (3) in the form of an obstruction to separate the reaction area (2) from the detection area (4) that comprises a channel.

2. The enzyme screening bioreactor (10) of claim 1, wherein the channel has an opening smaller than 100 micron.

3. A platform comprising a plurality of the screening bioreactors of any of the former claims for the high-throughput enzyme screening.
